# EUROPÄISCHE PATENTSCHRIFT

(11) **EP 3 664 923 B1**
(45) Veröffentlichungstag und Bekanntmachung des Hinweises auf die Patenterteilung: **23.10.2024**
(21) Anmeldenummer: 18737253.7
(22) Anmeldetag: 04.07.2018
(51) Int. Cl.: B01F 27/808, B01F 35/513, C12M 1/00, C12M 1/06, B01F 35/30

(54) **MISCHVORRICHTUNG UND VERFAHREN ZUM BETREIBEN EINER MISCHVORRICHTUNG**
MIXING APPARATUS AND METHOD FOR OPERATING A MIXING APPARATUS
MELANGEUR ET PROCEDE POUR FONCTIONNER UN MELANGEUR

(30) Priorität: 10.08.2017 DE 102017007557
(43) Veröffentlichungstag der Anmeldung: 17.06.2020
(73) Patentinhaber: Sartorius Stedim Biotech GmbH, 37079 Göttingen (DE)
(72) Erfinder: ZEUCH, Stefan, 37079 Göttingen (DE); HÄUSSER, Markus, 37073 Göttingen (DE); WEISSHAAR, Stefan, 37139 Adelebsen (DE); LUDWIG, Jens, 37127 Jühnde (DE)
(74) Vertreter: Novagraaf International SA
(86) Internationale Anmeldenummer: PCT/EP2018/068050
(87) Internationale Veröffentlichungsnummer: WO 2019/029913

(56) Entgegenhaltungen:
- WO-A1-2016/179236
- DE-U1- 202006 014 665

## Beschreibung

Die Erfindung betrifft eine Mischvorrichtung und ein Verfahren zum Betreiben einer Mischvorrichtung.

Mischvorrichtungen für zum Beispiel Bioreaktoren, Mischbeutel und/oder Pallettanks dienen zur Aufnahme, zur Lagerung und/oder zum Mischen von Fluiden und/oder Feststoffen wie z.B. biologischen Medien. Dabei können biologische Medien in Mischbehältern wie z.B. Beuteln bereitgestellt werden. Die biologischen Medien werden innerhalb eines solchen Mischbehälters in die Mischvorrichtung eingebracht, in dem sie gelagert, temperiert und/oder durchmischt werden können. In einer solchen Mischvorrichtung können unterschiedliche Untersuchungen an dem biologischen Medium vorgenommen werden.

Dabei kann die Handhabung des Mischbehälters in einer sterilen Umgebung erfolgen. Das Mischen des Fluids und/oder Feststoffs kann dabei mittels eines rotierenden Rührelements erfolgen, das in den Mischbehälter hineingesteckt wird und von außerhalb des Mischbehälters angetrieben wird. Dabei kann das medienberührende Rührelement zu einer Rotation angetrieben werden. Der Antrieb für das Rührelement, wie z.B. ein Motor, sollte dabei nicht mit dem Medium in Kontakt kommen, um nicht kontaminiert zu werden und für einen nachfolgenden Prozess nicht gereinigt und/oder sterilisiert werden zu müssen. An einer besonders kritischen Stelle jedoch kann eine Antriebswelle von außen in das Innere des Mischbehälters eingeführt werden, um das darin befindliche Medium rührend durchmischen zu können. Dabei ist es technisch aufwendig, eine Antriebswelle, die um ihre Längsachse rotiert, sicher und steril so in das Innere des Behälters einzuführen, dass das Innere des Behälters auch unter einer Rotationsbewegung der Antriebswelle dicht bleibt gegenüber dem Äußeren des Mischbehälters.

Das Dokument WO 2016/179236 A1 offenbart eine Dichtungsanordnung, die einen Dichtungskörper mit einem darin ausgebildeten Raum aufweist. Ein Dichtungsträger wird in dem Raum gehalten, wobei der Dichtungsträger selektiv zwischen einer Dichtungsposition und einer Rotationsposition positionierbar ist, indem er sich entlang eines Transportwegs bewegt. Mindestens eine Dichtung wird von dem Dichtungsträger getragen. Eine hermetische Dichtung wird innerhalb des Raums außerhalb des Transportwegs des Dichtungsträgers gehalten, wobei die hermetische Dichtung konfiguriert ist, von einem rotierenden Element, gegen das die hermetische Dichtung drückt, abgescheuert zu werden.

Das Dokument DE 20 2006 014 665 U1 offenbart eine Dispergiervorrichtung mit einem Motor. Eine Welle mit einem Rührwerkzeug wird durch den Motor angetriebenen. Ein Behälter dient zur Aufnahme eines Dispergierguts und des Rührwerkzeugs. Ein Dichtungssystem dient zum Abdichten des Behälters gegenüber der Welle. Das Dichtungssystem umfasst ein für die Dauer einer Evakuierung aus einer Ruheposition in eine Dichtungsposition zuschaltbares Dichtungselement.

Der Erfindung liegt die Aufgabe zugrunde, ein verbessertes Mischsystem zu ermöglichen, das insbesondere eine sichere Einführung der Antriebskraft in das Fluid und/oder den Feststoff ermöglicht.

Diese Aufgabe wird durch die Gegenstände der unabhängigen Ansprüche gelöst. Bevorzugte Ausführungsformen sind die Gegenstände der abhängigen Ansprüche. Ein Aspekt betrifft eine Mischvorrichtung zum Mischen eines Fluids und/oder Feststoffs mit einem Mischbehälter, gemäß dem Gegenstand des Anspruch 1, in dessen Inneren das Fluid und/oder der Feststoffs anordenbar ist. Der Mischbehälter kann in einer Mischbehälteraufnahme der Mischvorrichtung angeordnet sein. Die Mischvorrichtung weist eine Durchführung durch eine Behälterwand des Mischbehälters auf und eine durch die Durchführung geführte Antriebswelle zum Antreiben eines zumindest teilweise im Inneren des Mischbehälters angeordneten Rührelements zum Mischen des im Mischbehälter angeordneten Fluids und/oder Feststoffs. Ein antriebseitiges Wellenende der Antriebswelle ist zum Ankoppeln eines außerhalb des Mischbehälters angeordneten Antriebs an die Antriebswelle ausgebildet. Die Mischvorrichtung weist eine Einstelldichtung zum Abdichten der Durchführung auf, wobei die Einstelldichtung in zumindest zwei Dichtbetriebszustände mit unterschiedlichen Dichtwirkungen einstellbar ist.

Die Mischvorrichtung kann insbesondere zur Durchmischung eines Mischbehälters ausgebildet sein, welcher als Bioreaktorbeutel, Pallettank und/oder als ein anderer Mischbeutel der eingangs beschriebenen Art ausgebildet sein kann. Die Mischvorrichtung kann neben dem Mischbehälter auch eine Mischbehälteraufnahme für den Mischbehälter aufweisen, der zum Aufnehmen des Fluids und/oder des Feststoffs ausgebildet ist. Die Mischbehälteraufnahme kann eine Lagerung, Ankoppelung und/oder einen Anschluss zum Lagern, Ankoppeln und/oder Anschließen des Mischbehälters aufweisen. Insbesondere kann an der Mischbehälteraufnahme ein Einlass und/oder ein Ablass zum Anschließen an den Mischbehälter ausgebildet sein. Weiterhin kann die Durchführung so am Mischbehälter ausgebildet sein, dass die Antriebswelle durch die Durchführung hindurch ins Innere des Mischbehälters anordenbar ist. Die Mischvorrichtung kann zur Aufnahme des Mischbehälters die Mischbehälteraufnahme aufweisen, in die der Mischbehälter so aufgenommen werden kann, dass z.B. ein antriebseitiges Ende der Antriebswelle mit einem außerhalb des Mischbehälters angeordneten Antrieb gekoppelt werden kann. Die Mischvorrichtung kann an Stelle der Mischbehälteraufnahme den Mischbehälter aufweisen. Insbesondere kann die Mischvorrichtung auch lediglich aus dem Mischbehälter bestehen, also als der Mischbehälter ausgebildet sein.

Das Fluid und/oder der Feststoff kann als biologisches Medium ausgebildet sein. Weiterhin kann der Mischbehälter auch zur Aufnahme eines Fluidgemischs und/oder eines Feststoffgemischs ausgebildet sein und/oder ein solches Gemisch enthalten.

Der Mischbehälter kann an und/oder in der Mischbehälteraufnahme der Mischvorrichtung angeordnet sein. Der Mischbehälter kann zumindest teilweise als flexibler Beutel ausgebildet sein, also eine flexible Beutelwand aufweisen. Alternativ oder zusätzlich kann der Mischbehälter zumindest teilweise im Wesentlichen steife und/oder starre Behälterwände aufweisen, die zum Beispiel metallisch oder aus einem Hartkunststoff ausgebildet sein können. Der Mischbehälter kann als ein sogenannter "single-use-bag" ausgebildet sein, also als ein Einwegbeutel, der nach dem Mischvorgang entsorgt werden kann. Der Mischbehälter enthält das Fluid und/oder den Feststoff, das bzw. der vermischt wird.

Zum Mischen des Fluids und/oder des Feststoffs dient eine Bewegung des Rührelements, z.B. eine Rotationsbewegung. Die Rotationsbewegung des Rührelements kann um eine Rotationsachse erfolgen, wobei die Rotationsachse so angeordnet ist, dass die Rotationsachse durch das Rührelement hindurch verläuft, z.B. durch einen Mittelpunkt und/oder Schwerpunkt des Rührelements.

Zum Antreiben des Rührelements dient der Antrieb, der als ein Motor ausgebildet sein kann, insbesondere ein elektrischer Motor, der außerhalb des Mischbehälters und somit nicht medienberührend angeordnet sein kann. Der Antrieb kann als Bestandteil der Mischvorrichtung ausgebildet sein. Alternativ kann die Mischvorrichtung selbst antriebslos ausgebildet sein und lediglich zum Anschließen des Antriebs an die Antriebswelle ausgebildet sein.

Die Antriebskraft des Antriebs wird dabei zumindest teilweise über die Antriebswelle vom Antrieb auf das Rührelement übertragen. Mit anderen Worten treibt der Antrieb die Antriebswelle z.B. zu einer Rotationsbewegung um eine Symmetrieachse der Antriebswelle an. Die Bewegung der Antriebswelle ist auf das Rührelement übertragbar.

Die Antriebswelle kann so angeordnet sein, dass sie von außerhalb des Mischbehälters zumindest teilweise in das Innere des Mischbehälters hinein weisend angeordnet ist. Das außerhalb und/oder am Rand des Mischbehälters angeordnete Ende der Antriebswelle ist als das antriebsseitige Wellenende ausgebildet, welches direkt oder indirekt mit dem Antrieb verbunden sein kann und/oder verbunden werden kann. Ein gegenüberliegendes Ende der Antriebswelle, das in das Innere des Mischbehälters hinein weist, insbesondere ein Ende, das im Inneren des Mischbehälters angeordnet ist, ist direkt oder indirekt (z.B. über eine Lagerung und/oder Kupplung) mit dem Rührelement verbunden. Dieses Ende wird als das rührseitige Wellenende bezeichnet. Bevorzugt ist das rührseitige Wellenende starr und/oder direkt mit dem Rührelement verbunden, wodurch der Bauteilaufwand reduziert wird, da z.B. keine zusätzliche Kupplung notwendig ist.

Die Antriebswelle kann im Wesentlichen länglich ausgebildet sein, z.B. in Form eines Zylinders, dessen Zylinderachse um ein Vielfaches größer ist als dessen Durchmesser. Der Umfang des Zylinders kann zumindest teilweise rund und/oder zumindest teilweise kantig bzw. eckig ausgebildet sein. Die Antriebswelle kann zumindest teilweise so angeordnet sein, dass die Zylinderachse der Rührstange mit der Rotationsachse zusammenfällt, um die die Rotationsbewegung des Rührelements erfolgt.

Die Antriebswelle ist an der Durchführung durch die Behälterwand des Mischbehälters geführt. Die Durchführung kann als eine Öffnung und/oder eine Aussparung ausgebildet sein. Die Durchführung kann verstärkt ausgebildet sein, also z.B. ein Gehäuse aufweisen, welches die Durchführung zumindest teilweise umgibt und/oder definiert. Mit anderen Worten weist die Behälterwand, und gegebenenfalls auch die Mischbehälteraufnahme, eine Durchführung durch die Behälterwand auf, deren Innendurchmesser zumindest so groß ist wie der Außendurchmesser der Antriebswelle. Die Durchführung kann hierbei insbesondere an einem unteren Bereich des Mischbehälters angeordnet sein. Zum Beispiel kann die Durchführung zentral unterhalb der Mischbehälteraufnahme und/oder des Mischbehälters angeordnet sein. Allgemein kann es erforderlich sein, an der Durchführung Dichtungen wie z.B. einen O-Ring vorzusehen, um sowohl eine Kontamination des Inhalts des Mischbehälters zu reduzieren als auch ein Austreten des Fluids oder des Feststoffs aus dem Mischbehälter.

An der Durchführung kann eine Mehrzahl von Dichtungen ausgebildet und/oder vorgesehen sein. Eine der Dichtungen ist als die Einstelldichtung ausgebildet. Die Einstelldichtung kann die Durchführung in den zumindest zwei unterschiedlichen Dichtbetriebszuständen abdichten. Dabei ist die Einstelldichtung von einem ersten Dichtbetriebszustand in einen zweiten Dichtbetriebszustand einstellbar und umgekehrt. Mit anderen Worten ist die Einstelldichtung wiederholt einstellbar, z.B. reversibel zwischen zwei verschiedenen Dichtbetriebszuständen.

Die Einstelldichtung kann hierbei z.B. mechanisch und/oder elektrisch verstellbar sein. Die Einstellbarkeit der Einstelldichtung ermöglicht eine Anpassung der Dichtwirkung der Einstelldichtung, z.B. auf den Betriebszustand des Antriebs und/oder als eine Nachjustage aufgrund von Alterungsvorgängen. Das Vorsehen einer einstellbaren Einstelldichtung ermöglicht eine Anpassung der Dichtwirkung auf die aktuell benötigte Dichtwirkung in der Mischvorrichtung.

Gemäß einer Ausführungsform dichtet die Einstelldichtung in einem Dichtzustand als erster Dichtbetriebszustand die Durchführung ab und in einem Öffnungszustand als zweiter Dichtbetriebszustand ist die Einstelldichtung geöffnet und dichtet die Durchführung nicht ab. Mit anderen Worten ist die Einstelldichtung zwischen dem Dichtzustand und dem Öffnungszustand (z.B. reversibel) einstellbar. Dies kann dazu genutzt werden, die Einstelldichtung lediglich während eines ersten Betriebszustands des Anstriebsmotors in den Öffnungszustand einzustellen, z.B. wenn der Antrieb die Antriebswelle und damit das Rührelement antreibt. Ist der Antrieb in einem zweiten Betriebszustand ausgeschaltet und/oder treibt der Antrieb die Antriebswelle gerade nicht an, kann die Einstelldichtung in den Dichtzustand eingestellt werden. Die Einstelldichtung kann in diesem Fall als eine im Wesentlichen statische Dichtung verwendet werden, die die Durchführung lediglich dann abdichtet, wenn der Antrieb ruht. Dadurch wird die Einstelldichtung beim Antreiben des Rührelements durch die Rotationsbewegung der Antriebswelle nicht belastet und daher weniger stark strapaziert. Dies kann den Verschleiß der Einstelldichtung reduzieren. Zudem ermöglicht die Einstelldichtung einen besonders guten Abdichtungsschutz in Ruhezuständen der Mischvorrichtung, z.B. gegen Staub und/oder gegen das Eindringen von Reinigungsmitteln beim Reinigen der Mischvorrichtung. Zudem kann die Einstelldichtung auch in umgekehrter Richtung gegen den Austritt des Mediums aus dem Mischbehälter (Beutel) in die Umgebung (z.B. in einen Reinraum) abdichten. Die Einstelldichtung kann steifer und/oder mit einer stärkeren Dichtwirkung ausgebildet werden als gegebenenfalls zusätzlich vorhandene Dichtungen, die die Antriebswelle und die Durchführung auch im angetriebenen und somit bewegten Betriebszustand abzudichten haben.

Gemäß einer Ausführungsform liegt die Einstelldichtung im Dichtzustand eng an der Antriebswelle an und ist in dem Öffnungszustand zumindest teilweise von der Antriebswelle beabstandet. Dabei kann die Einstelldichtung im Öffnungszustand im Wesentlichen kontaktlos zur Antriebswelle angeordnet sein. Dadurch wird die Einstelldichtung einerseits im Betrieb durch eine Bewegung der Antriebswelle weniger stark durch deren Bewegung belastet, andererseits bremst die Einstelldichtung die Bewegung der Antriebswelle auch weniger im Betriebszustand der Mischvorrichtung.

In einer Weiterbildung der Ausführungsform ist die Einstelldichtung im Öffnungszustand vollständig beabstandet von der Antriebswelle angeordnet. Dies bedeutet, dass die Einstelldichtung im Öffnungszustand die Antriebswelle an keiner Stelle physikalisch kontaktiert. Hierdurch wird eine besonders gute Entlastung der Einstelldichtung im Mischbetrieb der Mischvorrichtung ermöglicht. Um die Einstelldichtung hinreichend von der Antriebswelle zu beabstanden, kann es ausreichend sein, einen schmalen Spalt zwischen der Antriebswelle und der Einstelldichtung zu öffnen. Dieser Spalt kann z.B. lediglich einen einzigen Millimeter breit sein. Bevorzugt ist der Spalt jedoch zumindest zwei Millimeter breit, um auch bei einer leichten Unwucht der Antriebswelle eine hinreichende Beabstandung gewährleisten zu können.

Gemäß einer Ausführungsform ist die Einstelldichtung dazu ausgebildet und vorgesehen, den Dichtzustand einzunehmen, wenn der Antrieb die Antriebswelle nicht antreibt, und den Öffnungszustand einzunehmen, wenn der Antrieb die Antriebswelle antreibt. In dieser Ausführungsform ist die Einstelldichtung sozusagen als eine stationäre Dichtung ausgebildet, die die Durchführung in unangetriebenen Ruhephasen der Mischvorrichtung abdichtet.

Allgemein kann die Einstelldichtung z.B. als ein Elastomer ausgebildet sein, der im Öffnungszustand zumindest teilweise von der Antriebswelle weggedrückt wird und im Dichtzustand an der Antriebswelle anliegt, bevorzugt von allen radialen Außenseiten.

Gemäß einer Ausführungsform weist die Mischvorrichtung eine Einstellvorrichtung zum Einstellen des Dichtbetriebszustands der Einstelldichtung auf. Eine Betätigung der Einstellvorrichtung kann ein Wechsel des Dichtbetriebszustands der Einstelldichtung bewirken. Die Änderung des Dichtbetriebszustands kann reversibel sein und wiederholt erfolgen. Die Einstellvorrichtung kann ein Betätigungselement aufweisen zur Betätigung der Einstellvorrichtung. Die Einstellvorrichtung kann insbesondere an und/oder in der Durchführung und/oder benachbart zur Einstelldichtung ausgebildet sein.

In einer Weiterbildung dieser Ausführungsform ist die Einstellvorrichtung in Achsrichtung der Antriebswelle verschiebbar gelagert. Dies ermöglicht ein Verschieben der Einstellrichtung in Achsrichtung, z.B. näher zur Einstelldichtung hin und/oder weiter von der Einstelldichtung weg. Dadurch wird eine besonders günstige Betätigung der Einstellung der Einstelldichtung ermöglicht.

In einer Weiterbildung der Ausführungsform ist die Einstellvorrichtung im Wesentlichen ringförmig um die Antriebswelle herum ausgebildet. Dies ermöglicht eine großflächige Betätigung der Einstelldichtung, welche ebenfalls im Wesentlichen ringförmig um die Antriebswelle herum ausgebildet sein kann. Die Einstellvorrichtung und/oder die Einstelldichtung kann insbesondere vollumfänglich um die Antriebswelle herum ausgebildet sein.

Gemäß einer Weiterbildung der Ausführungsform ist die Einstellvorrichtung im Wesentlichen konisch ausgebildet. Ein schmales Ende der Einstellvorrichtung weist in einem Dichtbetriebszustand mit hoher Dichtwirkung zur Einstelldichtung hin. Die konische Form bedeutet, dass die Einstellvorrichtung ein schmales Ende aufweist und ein breites Ende, welches z.B. zumindest etwa 120%, bevorzugt zumindest etwa 150%, breiter als das schmale Ende ausgebildet ist. Hierbei kann die konische Form der Einstelldichtung teilweise unterbrochen sein. Das schmale Ende der Einstellvorrichtung weist in dem Dichtbetriebszustand mit hoher Dichtwirkung zur Einstelldichtung hin. In diesem Dichtbetriebszustand mit hoher Dichtwirkung kann die Einstellvorrichtung beabstandet von der Einstelldichtung angeordnet sein, um so den physikalischen Kontakt zwischen der Einstelldichtung und der Antriebswelle zu ermöglichen. Wird die Einstellvorrichtung bei Betätigung mit ihrem schmalen Ende voran auf die Einstelldichtung zu bewegt, so dass die Einstellvorrichtung die Einstelldichtung kontaktiert, so kann die Einstelldichtung so bewegt werden, dass sich ihre Position in der Durchführung ändert, wobei die Dichtwirkung der Einstelldichtung ebenfalls geändert wird. Beispielsweise kann die Einstellvorrichtung mit ihrem schmalen Ende voran zwischen die Einstelldichtung und die Antriebswelle eingebracht werden. Alternativ oder zusätzlich kann die Einstellvorrichtung auch zwischen die Einstelldichtung und ein äußeres Gehäuse der Durchführung eingebracht werden, um die Dichtwirkung zu ändern. Bevorzugt wird die Einstellvorrichtung jedoch zum Reduzieren der Dichtwirkung zwischen die Einstelldichtung und die Antriebswelle eingebracht, um so die Einstelldichtung von der beweglichen Antriebswelle abzulösen. Dadurch kann der Verschleiß der Einstelldichtung im Dichtungsbetriebszustand mit geringer Dichtwirkung reduziert werden.

Erfindungsgemäß weist die Mischvorrichtung in der Durchführung einen Partikelabscheider auf. Partikelabscheider dienen zum Abscheiden unerwünschter Partikel. Ein Partikelabscheider stellt einen rotatorischen Partikelschutz bereit z.B. gegenüber einem Abrieb, welcher zwischen Radialwellendichtring und Welle und/oder Wälzlager(n) generiert wird. Der Partikelabscheider kann einen Partikeleintritt in den Mischbeutel/-tank und damit den Kontakt der Partikel zum Medium (Produkt) reduzieren und/oder verhindern. Der Partikelabscheider besteht vorzugsweise aus (z.B. thermoplastischem) Kunststoff und kann zwei Rückhaltelippen aufweisen. Der Partikelabscheider kann zumindest eine der beiden folgenden Funktionen erfüllen: Der Partikelabscheider kann das Innere des Mischbehälters gegenüber Partikeln aus der Durchführung, beispielsweise von einem Gehäuse an der Durchführung, schützen. Weiterhin kann der Partikelabscheider das Gehäuse vor Laugen, Säuren und/oder abrasiven Werkstoffen schützen, welche in dem Mischbehälter gemischt werden sollen. Der Partikelabscheider kann insbesondere in doppelter Funktion beide dieser Wirkungen aufweisen. Der Partikelabscheider kann außerdem mit einer Partikelabscheiderdichtung wie z.B. einem O-Ring in der Durchführung befestigt sein. Der O-Ring kann radial zwischen dem Partikelabscheider und einem Gehäuse der Durchführung angeordnet sein. Der Partikelabscheider kann um die Antriebswelle herum in der Durchführung angeordnet sein.

Erfindungsgemäß ist der Partikelabscheider an einem behälterseitigen und/oder rührseitigen Durchführungsende der Durchführung angeordnet. Mit anderen Worten kann der Partikelabscheider an dem Ende der Durchführung angeordnet sein, das dem Inneren des Mischbehälters zugewandt ist. So kann eine erste Seite des Partikelabscheiders in Kontakt mit dem Fluid und/oder dem Feststoff stehen, der oder das in der Mischvorrichtung gemischt werden soll, und mit einer zweiten Seite in Rotationsachsenrichtung von dem Inneren des Mischbehälters weg zeigen. Insbesondere an dieser exponierten Position in der Durchführung kann der Partikelabscheider die voranstehend beschriebene Doppelfunktion erfüllen.

Gemäß einer Ausführungsform ist die Einstelldichtung im Wesentlichen ringförmig und/oder vollumfänglich um die Antriebswelle herum angeordnet. Mit anderen Worten kann die Einstelldichtung von der Antriebswelle durchstoßen sein. In dieser Position kann die Einstelldichtung die Durchführung und/oder den Raumbereich zwischen der Durchführung und der Antriebswelle besonders günstig abdichten.

Gemäß einer Ausführungsform ist ein Gehäuse an der Durchführung angeordnet und die Antriebswelle im Inneren des Gehäuses beweglich gelagert, insbesondere drehbar. Das Gehäuse kann dabei die Antriebswelle zumindest teilweise umfassen. Zwischen der Antriebswelle und dem Gehäuse können eine oder mehrere Dichtungen und/oder Kugellager angeordnet sein. Insbesondere kann die Einstelldichtung im Inneren des Gehäuses angeordnet sein. Bevorzugt ist die Einstelldichtung an einem dem Mischbehälter abgewandten Ende des Gehäuses angeordnet. Dadurch kann die Einstelldichtung besonders gut vor äußeren Verschmutzungen der Mischvorrichtung schützen, wie z.B. Staub und/oder Reinigungsmitteln. An und/oder benachbart zu dem Gehäuse kann der Antrieb angeordnet sein, der die im Inneren des Gehäuses angeordnete Antriebswelle antreibt.

In einer Ausführungsform ist die Einstelldichtung zwischen der Antriebswelle und dem Gehäuse angeordnet und weist einen beweglichen Dichtbereich aufweist. Eine Bewegung des beweglichen Dichtbereichs kann eine Veränderung der Position und/oder Lage dieses beweglichen Dichtbereichs im Inneren des Gehäuses zur Folge haben. Die Bewegung kann eine Veränderung der Dichtwirkung der Einstelldichtung und somit eine Einstellung des Dichtbetriebszustands der Einstelldichtung bewirken. Der bewegliche Dichtbereich kann z.B. angrenzend und/oder benachbart zur Antriebswelle angeordnet sein. Der bewegliche Dichtbereich kann elastisch verformbar sein. Die Bewegung des beweglichen Dichtbereichs kann eine elastische Verformung umfassen, insbesondere ein zumindest teilweises Stauchen und/oder ein zumindest teilweises Dehnen des beweglichen Dichtbereichs. Zusätzlich kann die Einstelldichtung einen statischen Dichtbereich aufweisen, dessen Position und/oder Lage im Wesentlichen unveränderlich ist. Der statische Dichtbereich kann z.B. angrenzend an das Gehäuse ausgebildet sein.

In einer Weiterbildung dieser Ausführungsform weist das Gehäuse an einem antriebseitigen Ende der Durchführung eine Verstärkung auf. Zumindest an dieser Stelle ist das Gehäuse mechanisch verstärkt, z.B. durch einen Metallring, welcher z.B. von außen um das antriebseitige Gehäuse herum angeordnet ist. Die Verstärkung kann zumindest teilweise metallisch ausgebildet sein. Dadurch kann die mechanische Steifigkeit der Gehäusefläche in diesem besonders beanspruchten Bereich erhöht werden, da z.B. ein Gehäuse aus Kunststoff per se nicht immer eine ausreichend hohe mechanische Steifigkeit aufweist. Da an diesem antriebseitigen Bereich die z.B. bewegliche Einstelldichtung angeordnet sein kann, ist an diesem Gehäuseteil und auf diesen Gehäuseteil einwirkend die höchste mechanische Belastung zu erwarten.

In einer Ausführungsform weist die Mischvorrichtung zumindest eine zusätzliche Permanentdichtung auf, die unabhängig vom Dichtbetriebszustand der Einstelldichtung die Durchführung abdichtet. Diese zumindest eine Permanentdichtung gewährleistet als Dichtmittel eine Abdichtung der Durchführung selbst im z.B. geöffneten Zustand der Einstelldichtung, und gegebenenfalls auch in Dichtbetriebszuständen mit abgeschwächter und/oder geringer Dichtwirkung. Die Permanentdichtung kann dazu ausgelegt und vorgesehen sein, die Antriebswelle auch in einem angetriebenen Zustand, d.h. in einem bewegten Zustand, zu kontaktieren und dabei die Durchführung abzudichten.

Gemäß einer Ausführungsform weist die Mischvorrichtung den außerhalb des Mischbehälters angeordneten Antrieb zum Antreiben der Antriebswelle auf und/oder den Mischbehälter, in dessen Inneren das Fluid und/oder der Feststoff anordenbar ist.

Gemäß einer Ausführungsform weist die Mischvorrichtung den Mischbehälter auf, in dessen Inneren das Fluid und/oder Feststoff anordenbar ist. Dabei weist der Mischbehälter eine zumindest teilweise flexible Behälterwand auf und/oder ist der Mischbehälter als ein Bioreaktorbeutel und/oder Mischbeutel und/oder Pallettank ausgebildet. Der Mischbeutel kann insbesondere als ein Einwegbeutel ausgebildet sein, wie z.B. ein "single-use-bag". Dabei kann der Mischbehälter im Wesentlichen von allen Seiten eine flexible Behälterwand aufweisen, die z.B. aus einem flexiblen Kunststoff ausgebildet sein kann. Um die Durchführung herum kann der Mischbehälter eine Versteifung aufweisen und/oder eine Ankoppeleinrichtung zum Ankoppeln an die Mischbehälteraufnahme.

Ein Aspekt betrifft ein Verfahren zum Betreiben einer Mischvorrichtung zum Mischen eines Fluids oder Feststoffs gemäß Anspruch 17 mit den Schritten:
- Aufnehmen des Fluids und/oder Feststoffs in einen Mischbehälter;
- Mischen des im Mischbehälter angeordneten Fluids und/oder Feststoffs mittels eines zumindest teilweise im Inneren des Mischbehälters angeordneten Rührelements;
- Antreiben des Rührelements mittels einer Antriebswelle, welche von einem außerhalb des Mischbehälters angeordneten Antriebs angetrieben wird und welcher an einer Durchführung durch eine Behälterwand des Mischbehälters geführt ist; und
- Einstellen einer einstellbaren Einstelldichtung zum Abdichten der Durchführung in einen von zumindest zwei Dichtbetriebszuständen mit unterschiedlichen Dichtwirkungen.

Das Verfahren wird mittels einer Mischvorrichtung gemäß dem voranstehend beschriebenen Aspekt durchgeführt. Deswegen treffen alle im Zusammenhang mit der Mischvorrichtung gemachten Ausführungen auch auf das Verfahren zu und umgekehrt.

In einer Ausführungsform des Verfahrens wird die Einstelldichtung in einen Dichtzustand als erster Dichtbetriebszustand eingestellt, in welchem die Einstelldichtung die Durchführung abdichtet, insbesondere wenn die Antriebswelle nicht angetrieben wird. Weiterhin wird die Einstelldichtung in einen Öffnungszustand als zweiter Dichtbetriebszustand eingestellt, in welchem sie die Durchführung nicht und/oder reduziert abdichtet, insbesondere wenn die Antriebswelle angetrieben wird. In dieser Ausführungsform dient die Einstelldichtung z.B. als statische Abdichtung, die die Durchführung lediglich in einem Ruhezustand der Mischvorrichtung abdichtet und im Betrieb der Mischvorrichtung in den Öffnungszustand eingestellt wird.

Im Rahmen dieser Erfindung können die Begriffe "im Wesentlichen" und/oder "etwa" so verwendet sein, dass sie eine Abweichung von bis zu 5% von einem auf den Begriff folgenden Zahlenwert beinhalten, eine Abweichung von bis zu 5° von einer auf den Begriff folgenden Richtung und/oder von einem auf den Begriff folgenden Winkel.

Begriffe wie oben, unten, oberhalb, unterhalb, usw. beziehen sich - sofern nicht anders spezifiziert - auf das Bezugssystem der Erde in einer Betriebsposition des Gegenstands der Erfindung.

Die Erfindung wird nachfolgend anhand von in Figuren gezeigten Ausführungsbeispielen näher beschrieben. Hierbei können gleiche oder ähnliche Bezugszeichen gleiche oder ähnliche Merkmale der Ausführungsformen kennzeichnen. Einzelne in den Figuren gezeigte Merkmale können in anderen Ausführungsbeispielen implementiert sein. Es zeigen:
- Figur 1: in einer perspektivischen, schematischen Darstellung eine Mischvorrichtung zum Mischen eines Fluids und/oder Feststoffs mit einem Mischbehälter;
- Figur 2: in einer perspektivischen Darstellung eine Durchführung, ein Rührelement und einen Antrieb einer Mischvorrichtung;
- Figur 3: in einer perspektivischen Darstellung eine Durchführung und ein Rührelement einer Mischvorrichtung;
- Figur 4: in einem Querschnitt eine durch eine Behälterwand geführte Durchführung mit einem Rührelement einer Mischvorrichtung;
- Figur 5A: in einem Querschnitt eine Durchführung mit einer Einstelldichtung in einem Dichtzustand;
- Figur 5B: in einem Querschnitt die in Fig. 5A gezeigte Durchführung mit der Einstelldichtung in einem Dichtzustand; und
- Figur 5C: in einem Querschnitt die in Fig. 5A gezeigte Durchführung mit der Einstelldichtung in einem Öffnungszustand.

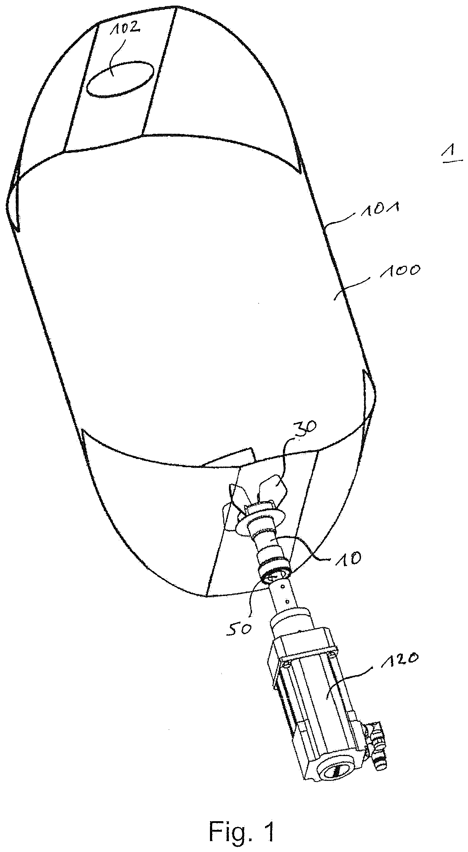

**Figur 1** zeigt in einer perspektivischen, schematischen Darstellung eine Mischvorrichtung 1 zum Mischen eines Fluids und/oder Feststoffs, welcher in einem Mischbehälter 100 angeordnet sein kann.

Die Mischvorrichtung 1 weist einen Antrieb 120 auf, welcher z.B. als ein Motor, insbesondere als ein Elektromotor, ausgebildet sein kann. Der Antrieb 120 treibt eine Antriebswelle 50 an, welche durch eine Durchführung 10 ins Innere des Mischbehälters 100 geführt ist. Der Antrieb 120 dient zum Antreiben eines Rührelements 30, welches im Inneren des Mischbehälters 100 angeordnet ist und dazu ausgebildet und vorgesehen ist, ein Medium im Inneren des Mischbehälters 100 zu durchmischen.

Der Antrieb 120, der Mischbehälter 100 und/oder das Rührelement 30 können als Bestandteil der Mischvorrichtung 1 ausgebildet sein. Alternativ kann eines, zwei oder alle drei dieser Bauteile als jeweils separates Bauteil ausgebildet sein, das nicht als Bestandteil der Mischvorrichtung 1 ausgebildet ist sondern lediglich zum Ankoppeln an die Mischvorrichtung 1.

Der Mischbehälter 100 kann als ein Mischbeutel und/oder als ein Bioreaktorbeutel ausgebildet sein. Der Mischbehälter 100 weist eine Behälterwand 101 auf, die zumindest teilweise aus einem flexiblen Material wie einem flexiblen Kunststoff ausgebildet sein kann. Der Mischbehälter 100 kann eine Einlassöffnung 102 aufweisen, durch die das Fluid und/oder der Feststoff ins Innere des Mischbehälters 100 eingefüllt und/oder ausgelassen werden kann. Im gezeigten Ausführungsbeispiel ist die Einlassöffnung 102 an einem Ende des Mischbehälters 100 angeordnet, welches gegenüberliegend zu der Durchführung 10 in der Behälterwand 101 ausgebildet ist.

In einem Ausführungsbeispiel kann die Durchführung 10 an einem unteren Ende des Mischbehälters 100 angeordnet sein. Die Einlassöffnung 102 kann z.B. an einem oberen Ende des Mischbehälters 100 angeordnet sein.

In einem Ausführungsbeispiel kann der Mischbehälter 100 auch als ein Pallettank zur Aufnahme eines Feststoffs ausgebildet sein.

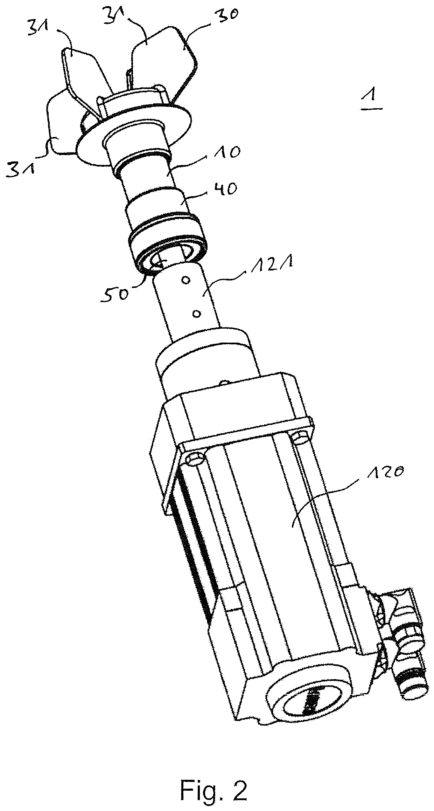

**Figur 2** zeigt in einer perspektivischen Darstellung die Durchführung 10, das Rührelement 30 sowie den Antrieb 120 der Mischvorrichtung 1. Der Antrieb 120 dient zum Antreiben einer Antriebswelle 50, von der in Figur 2 lediglich ein kleiner Abschnitt gezeigt ist. Der Antrieb 120 kann über eine Kupplung 121 mit der Antriebswelle 50 verbunden sein. Die Kupplung 121 kann insbesondere als eine Passfederkupplung ausgebildet sein. Der Antrieb 120 kann die Antriebswelle 50 z.B. zu einer Rotationsbewegung um ihre Längsachse antreiben.

Die Antriebswelle 50 ist im Inneren der Durchführung 10 angeordnet, welche an einer Öffnung in der Behälterwand 101 des Mischbehälters 100 ausgebildet ist. Die Durchführung 10 ist in dieser Ausführungsform als eine Wellendurchführung ausgebildet, innerhalb derer die Antriebswelle 50 durch die Behälterwand 101 hindurch geführt ist. Hierbei ist die Antriebswelle 50 so angeordnet, dass sie die Behälterwand 101 des Mischbehälters 100 durchstößt und/oder durchdringt. Die Durchführung 10 weist ein Gehäuse 40 auf, welches z.B. aus einem thermoplastischen Kunststoff ausgebildet sein kann. Die Antriebswelle 50 ist in dem Gehäuse 40 gelagert. Das Gehäuse 40 kann sowohl zur Lagerung der Antriebswelle 50 und/oder zur verbesserten Abdichtung ausgebildet und vorgesehen sein.

Das Rührelement 30 kann eine Mehrzahl von Rührflügeln 31 aufweisen, welche ins Innere des Mischbehälters 100 ragen. Im gezeigten Ausführungsbeispiel weist das Rührelement 30 vier Rührflügel 31 auf. In anderen Ausführungsformen kann das Rührelement 30 mehr oder weniger Rührflügel 31 aufweisen. Alternativ kann das Rührelement 30 auch eine andere Form aufweisen, z.B. schraubenförmig und/oder schüsselförmig ausgebildet sein.

Die Durchführung 10 ist als Verbindung zwischen dem Antrieb 120 und dem Rührelement 30 ausgebildet. In der gezeigten Ausführungsform ist die Durchführung 10 örtlich zwischen dem Rührelement 30 und dem Antrieb 120 angeordnet.

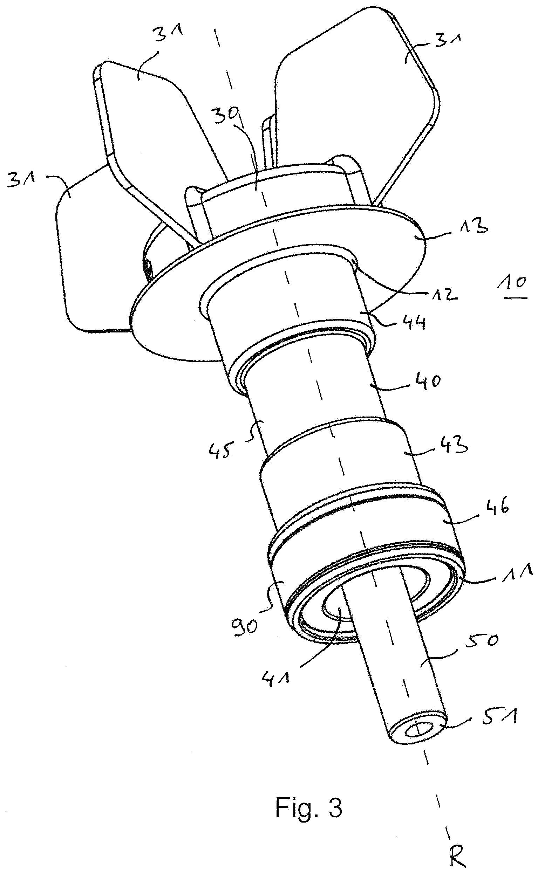

**Figur 3** zeigt die Durchführung 10 der Mischvorrichtung 1 mitsamt dem daran befestigten Rührelement 30 in einer perspektivischen Darstellung. Das Gehäuse 40 der Durchführung 10 ist im Wesentlichen rotationssymmetrisch ausgebildet bezüglich einer Rotationsachse R der Antriebswelle 50. Die Rotationsachse R der Antriebswelle 50 erstreckt sich entlang einer Längsachse und/oder Zylinderachse der Antriebswelle 50. Die Rotationsachse R der Antriebswelle 50 kann zugleich auch als die Rotationsachse des Rührelements 30 ausgebildet sein.

Ein antriebsseitiges Wellenende 51 ragt aus einer antriebsseitigen Gehäuseöffnung 41 am antriebsseitigen Durchführungsende 11 der Durchführung 10 aus dem Gehäuse 40 heraus. Das antriebsseitige Wellenende 51 ist dazu ausgebildet und vorgesehen, vom Antrieb 120 und/oder der Kupplung 121 kontaktiert zu werden. Insbesondere kann der Antrieb 120 über die Kupplung 121 mit dem antriebsseitigen Wellenende 51 gekoppelt werden.

Das Gehäuse 40 ist im Wesentlichen rotationssymmetrisch in Form eines Zylinders ausgebildet, dessen Zylinderachse mit der Rotationsachse R zusammenfällt. Das Gehäuse 40 kann im Querschnitt entlang einer senkrecht zur Rotationsachse R angeordneten Ebene im Wesentlichen kreisförmig ausgebildet sein, insbesondere im Wesentlichen entlang seiner gesamten Zylinderhöhe. Entlang der Rotationsachse R kann das Gehäuse 40 einmal oder mehrmals seinen (Zylinder-)Durchmesser ändern.

Das Gehäuse 40 weist einen mittleren Gehäusebereich 45 auf, welcher im gezeigten Ausführungsbeispiel den kleinsten Gehäusedurchmesser aufweist. Mit dem Gehäusedurchmesser wird hierbei die Ausdehnung des Gehäuses 40 senkrecht zur Rotationsachse R bezeichnet, also in radiale Richtung. Antriebsseitig und rührseitig an den mittleren Bereich 45 anschließend weist das Gehäuse 40 jeweils einen gegenüber dem mittleren Bereich 45 verbreiterten Bereich auf, d.h. einen Gehäusebereich mit einem gegenüber dem mittleren Bereich 45 verbreiterten Gehäusedurchmesser.

Antriebsseitig weist das Gehäuse 40 (z.B. angrenzend an den mittleren Gehäusebereich 45) einen ersten Gehäuseabsatz 43 auf, der einen breiteren Gehäusedurchmesser aufweist als der mittlere Gehäusebereich 45. Rührseitig weist das Gehäuse 40 einen zweiten Gehäuseabsatz 44 auf, der z.B. rührseitig an den mittleren Bereich 45 angrenzen kann und der im Vergleich zu jenem einen breiteren Gehäusedurchmesser aufweisen kann. Am Übergang des mittleren Gehäusebereichs 45 sowohl zum ersten Gehäuseabsatz 43 als auch zum zweiten Gehäuseabsatz 44 weist das Gehäuse 40 jeweils eine Gehäusestufe auf.

An einem rührseitigen und/oder behälterseitigen Durchführungsende 12 der Durchführung 10 ist ein Flansch 13 ausgebildet. Der Flansch 13 verstärkt die Behälterwand 101 des Mischbehälters 100 und kann als Mischbehälteraufnahme und/oder Verstärkung ausgebildet sein. Der Flansch 13 kann als Bestandteil des Mischbehälters 100 ausgebildet sein und/oder als Bestandteil der Durchführung 10. Im gezeigten Ausführungsbeispiel erstreckt sich der zweite Gehäuseabsatz 44 mit im Wesentlichen konstantem Gehäusedurchmesser vom mittleren Gehäusebereich 45 bis zum rührseitigen Durchführungsende 12, an dem der Flansch 13 ausgebildet ist.

Der erste Gehäuseabsatz 43 erstreckt sich mit im Wesentlichen konstantem Gehäusedurchmesser vom antriebsseitigen Ende des mittleren Bereichs 45 bis zu einem dritten Gehäuseabsatz 46 des Gehäuses 40.

Der dritte Gehäuseabsatz 46 weist einen gegenüber dem ersten Gehäuseabsatz 43 verbreiterten Gehäusedurchmesser auf. Der dritte Gehäuseabsatz 46 erstreckt sich mit im Wesentlichen konstantem Gehäusedurchmesser vom antriebsseitigen Ende des ersten Gehäuseabsatzes 43 bis zum antriebsseitigen Ende der Durchführung 10. An diesem antriebsseitigen Durchführungsende 11 ist die antriebsseitige Gehäuseöffnung 41 ausgebildet und angeordnet, welche im Wesentlichen ringförmig um die Antriebswelle 50 herum ausgebildet ist.

Das Gehäuse 40 weist einen Hohlraum vom antriebsseitigen Durchführungsende 11 bis zum rührseitigen Durchführungsende 12 auf, in welchem die Antriebswelle 50 angeordnet ist und/oder in welchem sie verlegt ist. Hierbei kann die Antriebswelle 50 aus dem vom Gehäuse 40 umschlossenen Hohlraum hinausragen, und zwar sowohl antriebsseitig als auch rührseitig.

Zumindest teilweise um den dritten Gehäuseabsatz 46 herum kann eine Verstärkung 90 ausgebildet sein, insbesondere ein Verstärkungsring, welcher z.B. als metallischer Verstärkungsring ausgebildet sein kann. Die Verstärkung 90 ist dazu ausgebildet und vorgesehen, den dritten Gehäuseabsatz 46 mechanisch zu verstärken und/oder zu stabilisieren. Dafür kann die Verstärkung 90 ringförmig um die Rotationsachse R der Antriebswelle 50 herum ausgebildet sein und von radial außen um den dritten Gehäuseabsatz 46 des Gehäuses 40 herum ausgebildet sein.

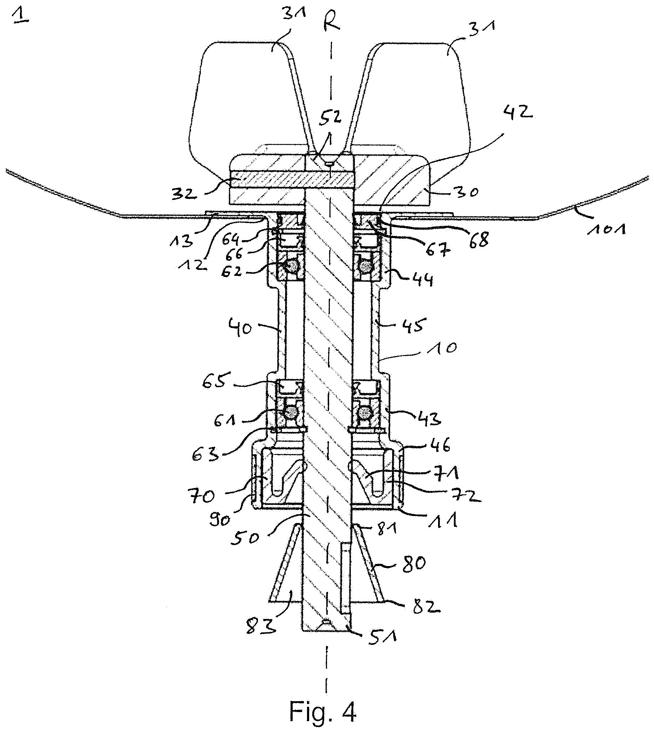

**Figur 4** zeigt einen Querschnitt durch die Mischvorrichtung 1 an der Durchführung 10.

Die Antriebswelle 50 ist im Wesentlichen stabförmig ausgebildet und erstreckt sich von dem antriebsseitigen Wellenende 51 bis zu ihrem rührseitigen Wellenende 52 entlang der Rotationsachse R. Dabei durchdringt die Antriebswelle 50 das Gehäuse 40 der Durchführung 10 vollständig vom antriebsseitigen Durchführungsende 11 bis zum rührseitigen Durchführungsende 12. Insbesondere durchdringt die Antriebswelle 50 dabei die Behälterwand 101 des in Figur 4 nur teilweise gezeigten Mischbehälters 100.

Am rührseitigen Wellenende 52 der Antriebswelle 50 ist die Antriebswelle 50 an das Rührelement 30 gekoppelt. Dazu ist ein Verbindungsmittel 32 vorgesehen, welches z.B. als eine Schraube ausgebildet sein kann. Das Verbindungsmittel 32 kann teilweise im Inneren der Antriebswelle 50 und teilweise im Inneren des Rührelements 30 ausgebildet und angeordnet sein und eine formschlüssige und/oder kraftschlüssige Verbindung zwischen dem Rührelement 30 und der Antriebswelle 50 bereitstellen.

Die Antriebswelle 50 ist im Inneren des Gehäuses 40 gelagert mittels zumindest einem Lager. Im gezeigten Ausführungsbeispiel ist die Antriebswelle 50 über ein erstes Lager 61 und ein zweites Lager 62 um die Rotationsachse R drehbar im Inneren des Gehäuses 40 gelagert. Die beiden Lager 61 und 62 sind in Ausbreitungsrichtung der Rotationsachse R voneinander beabstandet. Hierbei ist das erste Lager 61 im Inneren des ersten Gehäuseabsatzes 43 ausgebildet und das zweite Lager 62 im Inneren des zweiten Gehäuseabsatzes 44. Das erste Lager 61 und das zweite Lager 62 können jeweils als ein Rillenkugellager ausgebildet sein.

Das erste Lager 61 ist zwischen einer Gehäusestufe am Übergang vom mittleren Gehäusebereich 45 zum ersten Gehäuseabsatz 43 einerseits und einem Sprengring 63 andererseits so eingeklemmt, dass es entlang der Rotationsachse R im Wesentlichen unverschiebbar und/oder unbeweglich ist. Das zweite Lager 62 ist zwischen der Gehäusestufe am Übergang vom mittleren Gehäusebereich 45 zum zweiten Gehäuseabsatz 44 einerseits sowie einem zweiten Sprengring 64 andererseits so eingeklemmt, dass es entlang der Rotationsachse R im Wesentlichen unverschiebbar und/oder unbeweglich ist.

An der Seite des jeweiligen Lagers 61 und 62, die dem Rührelement 30 zugewandt ist, ist jeweils ein Dichtmittel ausgebildet. So ist zwischen dem ersten Lager 61 und der Gehäusestufe des Gehäuses 40 ein erstes Dichtmittel 65 angeordnet. Zwischen dem zweiten Lager 62 und dem zweiten Sprengring 64 ist ein zweites Dichtmittel 66 angeordnet. Das erste Dichtmittel 65 und/oder das zweite Dichtmittel 66 kann jeweils als eine Permanentdichtung ausgebildet sein, insbesondere als ein Radialwellendichtring. Das erste und/oder zweite Dichtmittel 65 und/oder 66 ist jeweils als eine dynamische Dichtung ausgebildet. Dies bedeutet, dass das jeweilige Dichtmittel 65 bzw. 66 sowohl an beweglichen als auch unbeweglichen Teilen der Mischvorrichtung 1 abdichtend anliegt. Hier sind sowohl das erste Dichtmittel 65 als auch das zweite Dichtmittel 66 zwischen der beweglichen Antriebswelle 50 und dem Gehäuse 40 angeordnet, wobei sie diese beiden Bauteile der Mischvorrichtung 1 gegeneinander abdichten. Das erste Dichtmittel 65 und das zweite Dichtmittel 66 dichten die Durchführung 10 zwischen der Antriebswelle 50 und dem Gehäuse 40 dynamisch ab, z.B. gegenüber einem Austreten des Mediums aus dem Mischbehälter 100 während des Mischvorgangs.

Das Gehäuse 40 kann aus einem thermoplastischen Kunststoff ausgebildet sein, welches über den Flansch 13 mit dem Mischbehälter 100 verschweißt sein kann, welcher ebenfalls aus Kunststoff ausgebildet sein kann.

Am rührseitigen Durchführungsende 12 ist eine rührseitige Gehäuseöffnung 42 ausgebildet, aus welcher die Antriebswelle 50 entlang der Rotationsachse R aus dem Gehäuse 40 hervorragt. Am rührseitigen Durchführungsende 12, insbesondere in und/oder an der rührseitigen Gehäuseöffnung 42, ist ein Partikelabscheider 67 ausgebildet, welcher mittels einer Partikelabscheiderdichtung 68 abgedichtet sein kann. Die Partikelabscheiderdichtung 68 kann z.B. als ein O-Ring ausgebildet sein und ist in radialer Richtung zwischen dem Partikelabscheider 67 und dem Gehäuse 40 angeordnet, insbesondere einem rührseitigen Ende des zweiten Gehäuseabsatzes 44. Der Partikelabscheider 67 ist Z kann dazu ausgebildet und vorgesehen einige oder sämtliche Partikel, die innerhalb des Gehäuses 40 während des Betriebs entstehen, innerhalb des Gehäuses 40 und somit außerhalb des Mischbehälters 100 zu belassen. Der Partikelabscheider 67 kann außerdem dazu ausgebildet und vorgesehen sein, eine Barriere und einen Schutz zu bilden für das zweite Dichtmittel 66, insbesondere gegenüber Laugen, Säuren und/oder abrasiven Werkstoffen aus dem Inneren des Mischbehälters 100.

Der erste Gehäuseabsatz 43, das erste Lager 61, der erste Sprengring 63 und das erste Dichtmittel 65 können jeweils als antriebseitiges Bauteil der Durchführung 10 ausgebildet sein, nämlich als antriebseitiger Gehäuseabsatz 43, als antriebseitiges Lager 61, als antriebseitiger Sprengring 63 und/oder als antriebseitiges Dichtmittel 65. Das zweite Lager 62, der zweite Gehäuseabsatz 44, der zweite Sprengring 64 und/oder das zweite Dichtmittel 66 kann als jeweils rührseitiges Bauteil der Durchführung 10 ausgebildet sein, nämlich als rührseitiger Gehäuseabsatz 44, als rührseitiges Lager 62, als rührseitiger Sprengring 64 und/oder als rührseitiges Dichtmittel 66.

Durch das Gehäuse 40 ist die Antriebswelle 50 geführt, welche aus einem Kunststoff ausgebildet sein kann. Am rührseitigen Wellenende 52 ist das Rührelement 30 ausgebildet, welches ebenfalls aus Kunststoff ausgebildet sein kann. Das Verbindungsmittel 32 dient zum mechanischen Verbinden der Antriebswelle 50 mit dem Rührelement 30. Am gegenüberliegenden Ende der Antriebswelle 50, nämlich dem antriebsseitigen Wellenende 51, ist die Antriebswelle 50 über die Kupplung 121 formschlüssig mit dem Antrieb 120 verbunden.

Das Gehäuse 40, ein Großteil der Antriebswelle 50, die Kupplung 121 und der Antrieb 120 sind außerhalb des Mischbehälters 100 angeordnet. Hierbei grenzt das Gehäuse 40 zumindest an den Mischbehälter 100 oder an die Mischbehälteraufnahme an. Das Gehäuse 40 der Durchführung 10 kann somit zumindest teilweise ins Innere des Mischbehälters 100 hineinragen. Der Flansch 13 kann z.B. innerhalb des Mischbehälters 100 angeordnet sein.

Angrenzend an das antriebsseitige Durchführungsende 11 ist der dritte Gehäuseabsatz 46 des Gehäuses 40 ausgebildet. Im gezeigten Ausführungsbeispiel weist der dritte Gehäuseabsatz 46 einen im Wesentlichen konstanten Gehäusedurchmesser auf, welcher größer ausgebildet ist als der Gehäusedurchmesser des ersten Gehäuseabsatzes 43 und/oder des zweiten Gehäuseabsatzes 44, und damit auch größer als der Gehäusedurchmesser des mittleren Gehäusebereichs 45.

Im Inneren des dritten Gehäuseabsatzes 46, zwischen einer Gehäusewand des Gehäuses 40 und der Antriebswelle 50, ist eine Einstelldichtung 70 ausgebildet. Die Einstelldichtung 70 kann als eine statische Dichtung ausgebildet sein, insbesondere als eine statische Elastomerdichtung. Die Einstelldichtung 70 ist dazu ausgebildet und vorgesehen, den Mischbehälter 100 im Stillstand der Mischvorrichtung 1 abzudichten. Bei Betrieb der Mischvorrichtung 1, also beim Antreiben des Rührelements 30, kann die Einstelldichtung 70 über eine Einstellvorrichtung 80 von der Antriebswelle 50 abgelöst werden. Im Stillstand der Mischvorrichtung 1 kann die Einstelldichtung 70 wieder reversibel an die Antriebswelle 50 angekoppelt werden.

Die Einstelldichtung 70 kann im Wesentlichen ringförmig um die Rotationsachse Rund um die Antriebswelle 50 herum ausgebildet sein. Dazu kann die Einstelldichtung 70 einen statischen Dichtbereich 72 aufweisen, welcher sich von radial innen an das Gehäuse 40, genauer den dritten Gehäuseabsatz 46 schmiegt und/oder an diesen angrenzt. Dieser statische Dichtbereich 72 der Einstelldichtung 70 ist im Wesentlichen unbeweglich ausgebildet und verändert seine Form und/oder seine Position und/oder seinen Stauchungsgrad im Inneren des Gehäuses 40 im Wesentlichen nicht. Der statische Dichtbereich 72 kann im Wesentlichen rohr- und/oder schlauchförmig um die Rotationsachse R herum und beabstandet von dieser ausgebildet sein.

Im Wesentlichen angrenzend an den statischen Dichtbereich 72 ist ein beweglicher Dichtbereich 71 der Einstelldichtung 70 ausgebildet, welcher den statischen Dichtbereich 72 im in Figur 4 gezeigten Dichtbetriebszustand mit der Antriebswelle 50 verbindet. Dazu kann der bewegliche Dichtbereich 71 in der gezeigten Schnittdarstellung entlang einer Ebene, in welcher die Rotationsachse R angeordnet ist, im Wesentlichen schräg zur Rotationsachse R ausgebildet sein, also winklig zur Rotationsachse R. Der bewegliche Dichtbereich 71 verläuft im gezeigten Ausführungsbeispiel von der Antriebswelle 50 weg winklig zur Rotationsachse R bis zum Gehäuseende des Gehäuses 40 am antriebsseitigen Durchführungsende 11. Dort ist ein Dichtübergangsbereich zwischen dem beweglichen Dichtbereich 71 und dem statischen Dichtbereich 72 ausgebildet, welcher diese beiden Dichtbereiche 71 und 72 miteinander verbindet.

Um den dritten Gehäuseabsatz 46 herum ist die Verstärkung 90 ausgebildet. Die Verstärkung 90 kann z.B. als ein Metallring ausgebildet sein und auf diesen Teil des Gehäuses 40 aufgeschrumpft sein, wodurch dieser Gehäuseteil stabilisiert wird.

Die Einstellvorrichtung 80 kann als ein Entkopplungsmechanismus ausgebildet sein. Die Einstellvorrichtung 80 ist im Wesentlichen konisch um die Antriebswelle 50 herum ausgebildet. Dabei weist die Einstellvorrichtung 80 ein schmales Ende 81 auf, welches in der gezeigten Position dem antriebsseitigen Durchführungsende 11 zugewandt ist. Die Einstellvorrichtung 80 weist zudem ein weites Ende 82 auf, welches in der gezeigten Darstellungsform von der Durchführung 10 abgewandt ist. Das schmale Ende 81 ist als rührseitiges Ende der Einstellvorrichtung 80 ausgebildet, während das weite Ende 82 als antriebsseitiges Ende der Einstellvorrichtung 80 ausgebildet ist. Vom rührseitigen Ende 81 bis zum antriebsseitigen Ende 82 nimmt der Außendurchmesser der Einstellvorrichtung 80 zu, im gezeigten Ausführungsbeispiel nimmt er im Wesentlichen stetig zu.

Im Inneren weist die Einstellvorrichtung 80 einen Durchgang 83 auf, welcher als ein Hohlraum ausgebildet ist und welcher von der Antriebswelle 50 durchdrungen ist. Die Einstellvorrichtung 80 ist im Wesentlichen rotationssymmetrisch bezüglich der Rotationsachse R ausgebildet, und wie ein Ärmel und/oder eine Manschette um die Antriebswelle 50 herum angeordnet.

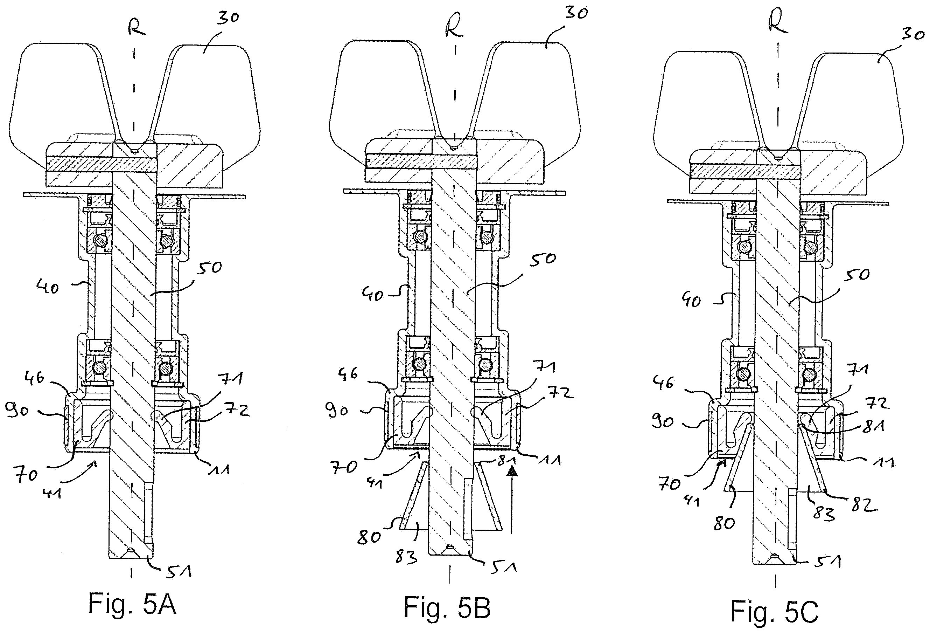

**Figuren 5A, 5B und 5C** zeigen in einem Querschnitt das Gehäuse 40 der Durchführung 10, wobei die Einstelldichtung 70 in unterschiedlichen Dichtbetriebszuständen eingestellt ist.

Figur 5A und 5B zeigen im Wesentlichen den bereits im Zusammenhang mit Figur 4 beschriebenen Zustand der Einstelldichtung 70. Dabei ist in Figur 5A die Einstellvorrichtung 80 nicht gezeigt. In Figur 5B ist gezeigt, wie die Einstellvorrichtung 80 in Pfeilrichtung, nämlich parallel zu Rotationsachse R auf das antriebsseitige Durchführungsende 11 und insbesondere die Einstelldichtung 70 zu, bewegt wird.

In der in den Figuren 5A und 5B gezeigten Ausführungsform liegt der bewegliche Dichtbereich 71 eng an der Antriebswelle 50 an. Hierbei wird das Innere des Gehäuses 40 und somit das Innere der Durchführung 10 gegenüber der äußeren Atmosphäre abgedichtet und umgekehrt. In der in Figur 5B gezeigten Situation ist die Einstelldichtung 70 in einen Dichtzustand als Dichtbetriebszustand eingestellt, indem es eine z.B. maximale Dichtwirkung ausübt und/oder aufweist. Hierbei ist die Einstellvorrichtung 80 kontaktlos zur Einstelldichtung 70, z.B. vollständig außerhalb des Gehäuses 40 angeordnet. Insbesondere ist die Einstellvorrichtung 80 und das schmale Ende 81 der Einstellvorrichtung 80 beabstandet von der Einstelldichtung 70 angeordnet, insbesondere beabstandet in Ausbreitungsrichtung der Rotationsachse R.

In Figur 5C ist die Einstellvorrichtung 80 ins Innere des Gehäuses 40 eingeschoben. Dabei dringt das rührseitige Ende 81 der Einstellvorrichtung 80 in das antriebsseitige Durchführungsende 11 ein. Das rührseitige Ende 81 der Einstellvorrichtung 80 ist zwischen dem beweglichen Dichtbereich 71 der Einstelldichtung 70 und der Antriebswelle 50 angeordnet. Dadurch öffnet sich ein Spalt zwischen der Einstelldichtung 70 und der Antriebswelle 50. Mit anderen Worten hält die Einstellvorrichtung 80 die Einstelldichtung 70 beabstandet von der Antriebswelle 50. In diesem Dichtbetriebszustand dichtet die Einstelldichtung 70 das Gehäuse 40 nicht mehr optimal ab, die Einstelldichtung 70 befindet sich in einem Öffnungszustand mit verringerter und reduzierter Dichtwirkung.

In dem Öffnungszustand der Einstelldichtung 70 weist der statische Dichtbereich 72 der Einstelldichtung 70 im Wesentlichen dieselbe Form, Position und Stauchung auf wie im Dichtzustand. Lediglich der bewegliche Dichtbereich 71 der Einstelldichtung 70 ist elastisch verformt durch mechanische Einwirkung der Einstellvorrichtung 80. Die Verformung der Einstelldichtung 70 erfolgt dabei elastisch und/oder reversibel. Mit anderen Worten stellt ein Herausbewegen der Einstellvorrichtung 80 entlang der Rotationsachse R aus dem dritten Gehäusebereich 46 wieder den in den Figuren 5A und Figur 5B gezeigten Dichtbetriebszustand her, in welchem die Einstelldichtung 70 das Innere des Gehäuses 40 abdichtet.

Die Bewegung der Einstellvorrichtung 80 kann mechanisch und/oder elektrisch erfolgen und/oder ausgelöst werden.

Die Einstelldichtung 70 kann als eine Dichtung mit besonders starker Dichtwirkung ausgebildet sein, insbesondere als Dichtung mit einer höheren Dichtwirkung als das erste und/oder zweite Dichtmittel 65 und/oder 66. Dadurch ist insbesondere in Ruhezuständen der Mischvorrichtung 1 eine besonders starke und/oder hohe Dichtwirkung erzielbar.

Eine solche starke Dichtwirkung kann z.B. von einem starken und/oder engen Anliegen der Einstelldichtung 70, insbesondere des beweglichen Dichtbereichs 71 an der Antriebswelle 50 bereitgestellt und/oder verursacht werden. Dies würde im Mischbetrieb die Antriebswelle 50 abbremsen und/oder zu einem starken Abrieb führen. Deswegen wird die Einstelldichtung 70 im Betrieb der Mischvorrichtung 1 mittels der Einstellvorrichtung 80 von der Antriebswelle 50 abgelöst, insbesondere beabstandet von ihr angeordnet. Im Betrieb dichten das erste Dichtmittel 65 und das zweite Dichtmittel 66 das Gehäuseinnere weiterhin gegenüber dem Fluid und/oder dem Feststoff im Inneren des Mischbehälters 100 ab. Im Öffnungszustand kann die Einstelldichtung 70 kontaktlos zur Antriebswelle 50 ausgebildet sein.

### Bezugszeichenliste

- 1: Mischvorrichtung

- 10: Durchführung
- 11: antriebseitiges Durchführungsende
- 12: behälterseitiges/rührseitiges Durchführungsende
- 13: Flansch

- 30: Rührelement
- 31: Rührflügel
- 32: Verbindungsmittel

- 40: Gehäuse
- 41: antriebseitige Gehäuseöffnung
- 42: rührseitige Gehäuseöffnung
- 43: erster Gehäuseabsatz
- 44: zweiter Gehäuseabsatz
- 45: mittlerer Gehäusebereich
- 46: dritter Gehäuseabsatz

- 50: Antriebswelle
- 51: antriebseitiges Wellenende
- 52: rührseitiges Wellenende

- 61: erstes Lager
- 62: zweites Lager
- 63: erster Sprengring
- 64: zweiter Sprengring
- 65: erstes Dichtmittel
- 66: zweites Dichtmittel
- 67: Partikelabscheider
- 68: Partikelabscheiderdichtung

- 70: Einstelldichtung
- 71: beweglicher Dichtbereich
- 72: statischer Dichtbereich

- 80: Einstellvorrichtung
- 81: schmales Ende
- 82: weites Ende
- 83: Durchgang

- 90: Verstärkung

- 100: Mischbehälter
- 101: Behälterwand
- 102: Einlassöffnung
- 120: Antrieb
- 121: Kupplung

- R: Rotationsachse

## Patentansprüche

1. Mischvorrichtung (1) zum Mischen eines Fluids und/oder Feststoffs mit:
- einem Mischbehälter (100), in dessen Inneren das Fluid und/oder Feststoff anordenbar ist;
- einer Durchführung (10) durch eine Behälterwand (101) des Mischbehälters (100);
- einer durch die Durchführung (10) geführten Antriebswelle (50) zum Antreiben eines zumindest teilweise im Inneren des Mischbehälters (100) angeordneten Rührelements (30) zum Mischen des im Mischbehälter (100) angeordneten Fluids und/oder Feststoffs;
- einem antriebseitigen Wellenende (51) der Antriebswelle (50) zum Ankoppeln eines außerhalb des Mischbehälters (100) angeordneten Antriebs (120) an die Antriebswelle (50);
- einer Einstelldichtung (70) zum Abdichten der Durchführung (10), wobei die Einstelldichtung (70) in zumindest zwei Dichtbetriebszustände mit unterschiedlichen Dichtwirkungen einstellbar ist;
**dadurch gekennzeichnet, dass** die Mischvorrichtung (1) einen in der Durchführung (10) angeordnetem Partikelabscheider (67) aufweist, wobei der Partikelabscheider (67) an einem behälterseitigen Durchführungsende (12) der Durchführung (10) angeordnet ist und zumindest einen rotatorischen Partikelschutz bereitstellt gegenüber einem Abrieb, welcher zwischen einer Radialwellendichtung (65; 66) und der Antriebswelle (50) generiert wird.

2. Mischvorrichtung nach Anspruch 1, wobei die Einstelldichtung (70) in einem Dichtzustand als erster Dichtbetriebszustand die Durchführung (10) abdichtet und die Einstelldichtung (70) in einem Öffnungszustand als zweiter Dichtbetriebszustand geöffnet ist und die Durchführung (10) nicht abdichtet.

3. Mischvorrichtung nach Anspruch 2, wobei die Einstelldichtung (70) im Dichtzustand eng an der Antriebswelle (50) anliegt und in dem Öffnungszustand zumindest teilweise von der Antriebswelle (50) beabstandet ist.

4. Mischvorrichtung nach Anspruch 2 oder 3, wobei die Einstelldichtung (70) im Öffnungszustand vollständig beabstandet von der Antriebswelle (50) angeordnet ist.

5. Mischvorrichtung nach einem der Ansprüche 2 bis 4, wobei die Einstelldichtung (70) dazu ausgebildet und vorgesehen ist:
- den Dichtzustand einzunehmen, wenn der Antrieb (120) die Antriebswelle (50) nicht antreibt, und
- den Öffnungszustand einzunehmen, wenn der Antrieb (120) die Antriebswelle (50) antreibt.

6. Mischvorrichtung nach einem der vorangegangenen Ansprüche, mit einer Einstellvorrichtung (80) zum Einstellen des Dichtbetriebszustands der Einstelldichtung (70).

7. Mischvorrichtung nach Anspruch 6, wobei die Einstellvorrichtung (80) in Achsrichtung (R) der Antriebswelle (50) verschiebbar gelagert ist.

8. Mischvorrichtung nach Anspruch 6 oder 7, wobei die Einstellvorrichtung (80) im Wesentlichen ringförmig um die Antriebswelle (50) herum ausgebildet ist.

9. Mischvorrichtung nach einem der Ansprüche 6 bis 8, wobei die Einstellvorrichtung (80) im Wesentlichen konisch ausgebildet ist und ein schmales Ende (81) der Einstellvorrichtung (80) in einem Dichtbetriebszustand mit hoher Dichtwirkung zur Einstelldichtung (70) hinweist.

10. Mischvorrichtung nach einem der vorangegangenen Ansprüche, wobei die Einstelldichtung (70) im Wesentlichen ringförmig um die Antriebswelle (50) angeordnet ist.

11. Mischvorrichtung nach einem der vorangegangenen Ansprüche, wobei ein Gehäuse (40) an der Durchführung (10) angeordnet ist und die Antriebswelle (50) im Inneren des Gehäuses (40) beweglich, insbesondere drehbar, gelagert ist.

12. Mischvorrichtung nach Anspruch 11, wobei die Einstelldichtung (70) zwischen der Antriebswelle (50) und dem Gehäuse (40) angeordnet ist und einen beweglichen Dichtbereich (71) aufweist.

13. Mischvorrichtung nach Anspruch 11 oder 12, wobei das Gehäuse (40) an einem antriebseitigen Ende der Durchführung (10) eine Verstärkung (90) aufweist.

14. Mischvorrichtung nach einem der vorangegangenen Ansprüche, mit zumindest einer zusätzlichen Permanentdichtung (65; 66), die unabhängig vom Dichtbetriebszustand der Einstelldichtung (70) die Durchführung (10) abdichtet.

15. Mischvorrichtung nach einem der vorangegangenen Ansprüche, mit dem außerhalb des Mischbehälters (100) angeordneten Antrieb (120) zum Antreiben der Antriebswelle (50) und/oder dem Mischbehälter (100), in dessen Inneren das Fluid und/oder Feststoff anordenbar ist.

16. Mischvorrichtung nach einem der vorangegangenen Ansprüche, mit dem Mischbehälter (100), in dessen Inneren das Fluid und/oder Feststoff anordenbar ist, wobei
- der Mischbehälter (100) eine zumindest teilweise flexible Behälterwand (101) aufweist und/oder
- wobei der Mischbehälter (100) als ein Bioreaktorbeutel und/oder Mischbeutel ausgebildet ist.

17. Verfahren zum Betreiben einer Mischvorrichtung nach einem der vorangegangenen Ansprüche zum Mischen eines Fluids und/oder Feststoffs mit den Schritten:
- Aufnehmen des Fluids und/oder Feststoffs in einen Mischbehälter (100);
- Mischen des im Mischbehälter (100) angeordneten Fluids und/oder Feststoffs mittels eines zumindest teilweise im Inneren des Mischbehälters (100) angeordneten Rührelements (30);
- Antreiben des Rührelements (30) mittels einer Antriebswelle (50), welche von einem außerhalb des Mischbehälters (100) angeordneten Antrieb (120) angetrieben wird und welche an einer Durchführung (10) durch eine Behälterwand (101) des Mischbehälters (100) geführt ist; und
- Einstellen einer einstellbaren Einstelldichtung (70) zum Abdichten der Durchführung (10) in einen von zumindest zwei Dichtbetriebszuständen mit unterschiedlichen Dichtwirkungen.

18. Verfahren nach Anspruch 17, wobei
- die Einstelldichtung (70) in einen Dichtzustand als erster Dichtbetriebszustand eingestellt wird, in welchem die Einstelldichtung (70) die Durchführung (10) abdichtet, insbesondere wenn die Antriebswelle (50) nicht angetrieben wird; und
- die Einstelldichtung (70) in einen Öffnungszustand als zweiter Dichtbetriebszustand eingestellt wird, in welchem sie die Durchführung (10) nicht und/oder reduziert abdichtet, insbesondere wenn die Antriebswelle (50) angetrieben wird.

## Claims

1. A mixing apparatus (1) for mixing a fluid and/or solid comprising:
- a mixing container (100), in the interior of which the fluid and/or solid can be arranged;
- a feed-through (10) through a container wall (101) of the mixing container (100);
- a drive shaft (50) guided through the feed-through (10) for driving a stirring element (30) arranged at least partially in the interior of the mixing container (100) for mixing the fluid and/or solid arranged in the mixing container (100);
- a drive-side shaft end (51) of the drive shaft (50) for coupling a drive (120) arranged outside of the mixing container (100) to the drive shaft (50);
- an adjustment seal (70) for sealing the drive-through (10), wherein the adjustment seal (70) can be set to at least two sealing operating states with different sealing effects;
**characterized in that** the mixing apparatus (1) has a particle separator (67) arranged in the feed-through (10), wherein the particle separator (67) is arranged at a containerside feed-through end (12) of the feed-through (10) and provides at least a rotary particle protection from abrasion, which is generated between a radial shaft seal (65; 66) and the drive shaft (50).

2. The mixing apparatus according to claim 1, wherein, in a sealing state as the first sealing operating state the adjustment seal (70) seals the feed-through (10) and, in an open state as the second sealing operating state the adjustment seal (70) is open and does not seal the feed-through (10).

3. The mixing apparatus according to claim 2, wherein, in the sealing state the adjustment seal (70) fits snugly on the drive shaft (50) and in the open state is at least partially spaced apart from the drive shaft (50).

4. The mixing apparatus according to claim 2 or 3, wherein, in the open state the adjustment seal (70) is arranged completely spaced apart from the drive shaft (50).

5. The mixing apparatus according to any of claims 2 to 4, wherein the adjustment seal (70) is configured and provided to:
- assume the sealing state when the drive (120) does not drive the drive shaft (50), and
- assume the open state when the drive (120) is driving the drive shaft (50).

6. The mixing apparatus according to any of the foregoing claims, comprising an adjustment apparatus (80) for adjusting the sealing operating state of the adjustment seal (70).

7. The mixing apparatus according to claim 6, wherein the adjustment apparatus (80) is displaceably pivoted in the axial direction (R) of the drive shaft (50).

8. The mixing apparatus according to claim 6 or 7, wherein the adjustment apparatus (80) is configured to be substantially annular around the drive shaft (50).

9. The mixing apparatus according to any of claims 6 to 8, wherein the adjustment apparatus (80) is configured to be substantially conical and in a sealing operating state, a narrow end (81) of the adjustment apparatus (80) points toward the adjustment seal (70) with a greater sealing effect.

10. The mixing apparatus according to any of the foregoing claims, wherein the adjustment seal (70) is arranged substantially in an annular manner around the drive shaft (50).

11. The mixing apparatus according to any of the foregoing claims, wherein a housing (40) is arranged on the feed-through (10) and the drive shaft (50) is movably, in particular rotatably, pivoted in the interior of the housing (40).

12. The mixing apparatus according to claim 11, wherein the adjustment seal (70) is arranged between the drive shaft (50) and the housing (40) and has a movable sealing region (71).

13. The mixing apparatus according to claim 11 or 12, wherein the housing (40) comprises a reinforcement (90) at a drive-side end of the feed-through (10).

14. The mixing apparatus according to any of the foregoing claims, comprising at least one additional permanent seal (65; 66) which seals the feed-through (10) independently of the sealing operating state of the adjustment seal (70).

15. The mixing apparatus according to any of the foregoing claims, comprising the drive (120) arranged outside of the mixing container (100) for driving the drive shaft (50) and/or the mixing container (100), in the interior of which the fluid and/or solid can be arranged.

16. The mixing apparatus according to any of the foregoing claims, comprising the mixing container (100) in the interior of which the fluid and/or solid can be arranged, wherein
- the mixing container (100) comprises an at least partially flexible container wall (101) and/or
- wherein the mixing container (100) is configured as a bioreactor bag and/or mixing bag.

17. A method for operating a mixing apparatus according to any of the foregoing claims for mixing a fluid and/or solid, comprising the steps of:
- receiving the fluid and/or solid in a mixing container (100) ;
- mixing the fluid and/or solid arranged in the mixing container (100) by means of a stirring element (30) arranged at least partially in the interior of the mixing container (100);
- driving the stirring element (30) by means of a drive shaft (50), which is driven by a drive (120) arranged outside of the mixing container (100) and which, at a feed-through (10) is guided through a container wall (101) of the mixing container (100); and
- setting an adjustable adjustment seal (70) for sealing the feed-through (10) to one of at least two sealing operating states comprising different sealing effects.

18. The method according to claim 17, wherein
- in a sealing state the adjustment seal (70) is set as a first sealing operating state, in which the adjustment seal (70) seals the feed-through (10), in particular when the drive shaft (50) is not being driven; and
- in an open state the adjustment seal (70) is set as a second sealing operating state, in which it does not seal the feed-through (10) and/or seals it to a lesser extent, in particular when the drive shaft (50) is being driven.

## Revendications

1. Dispositif de mélange (1) pour mélanger un fluide et/ou une matière solide comprenant :
- un contenant de mélange (100), à l'intérieur duquel le fluide et/ou la matière solide peut être disposé(e) ;
- un passage (10) à travers une paroi de contenant (101) du contenant de mélange (100) ;
- un arbre d'entraînement (50) guidé à travers le passage (10) pour entraîner un élément agitateur (30) disposé au moins en partie à l'intérieur du contenant de mélange (100) pour mélanger le fluide et/ou la matière solide disposé (e) dans le contenant de mélange (100) ;
- une extrémité d'arbre (51) côté d'entraînement de l'arbre d'entraînement (50) pour accoupler un entraînement (120) disposé à l'extérieur du contenant de mélange (100) à l'arbre d'entraînement (50) ;
- un joint de réglage (70) pour étanchéifier le passage (10), dans lequel le joint de réglage (70) peut être réglé dans au moins deux états de fonctionnement d'étanchéité avec différents effets d'étanchéité ;
**caractérisé en ce que** le dispositif de mélange (1) présente un séparateur de particules (67) disposé dans le passage (10), dans lequel le séparateur de particules (67) est disposé sur une extrémité de passage (12) côté contenant du passage (10) et fournit au moins une protection contre les particules rotative vis-à vis d'une abrasion, laquelle est générée entre un joint d'arbre radial (65 ; 66) et l'arbre d'entraînement (50).

2. Dispositif de mélange selon la revendication 1, dans lequel le joint de réglage (70) dans un état d'étanchéité comme premier état de fonctionnement d'étanchéité étanchéifie le passage (10) et le joint de réglage (70) dans un état d'ouverture comme deuxième état de fonctionnement d'étanchéité est ouvert et n'étanchéifie pas le passage (10).

3. Dispositif de mélange selon la revendication 2, dans lequel le joint de réglage (70) dans l'état d'étanchéité s'applique étroitement sur l'arbre d'entraînement (50) et dans l'état d'ouverture est espacé au moins en partie de l'arbre d'entraînement (50).

4. Dispositif de mélange selon la revendication 2 ou 3, dans lequel le joint de réglage (70) dans l'état d'ouverture est disposé de manière entièrement espacée de l'arbre d'entraînement (50) .

5. Dispositif de mélange selon l'une quelconque des revendications 2 à 4, dans lequel le joint de réglage (70) est réalisé et destiné à :
- occuper l'état d'étanchéité, lorsque l'entraînement (120) n'entraîne pas l'arbre d'entraînement (50), et
- occuper l'état d'ouverture, lorsque l'entraînement (120) entraîne l'arbre d'entraînement (50).

6. Dispositif de mélange selon l'une quelconque des revendications précédentes, comprenant un dispositif de réglage (80) pour régler l'état de fonctionnement d'étanchéité du joint de réglage (70).

7. Dispositif de mélange selon la revendication 6, dans lequel le dispositif de réglage (80) est monté de manière à pouvoir se déplacer dans la direction axiale (R) de l'arbre d'entraînement (50).

8. Dispositif de mélange selon la revendication 6 ou 7, dans lequel le dispositif de réglage (80) est réalisé de manière sensiblement annulaire autour de l'arbre d'entraînement (50).

9. Dispositif de mélange selon l'une quelconque des revendications 6 à 8, dans lequel le dispositif de réglage (80) est réalisé de manière sensiblement conique et une extrémité étroite (81) du dispositif de réglage (80) dans un état de fonctionnement d'étanchéité avec un effet d'étanchéité élevé est dirigée vers le joint de réglage (70).

10. Dispositif de mélange selon l'une quelconque des revendications précédentes, dans lequel le joint de réglage (70) est disposé de manière sensiblement annulaire autour de l'arbre d'entraînement (50).

11. Dispositif de mélange selon l'une quelconque des revendications précédentes, dans lequel un boîtier (40) est disposé sur le passage (10) et l'arbre d'entraînement (50) est monté mobile, en particulier rotatif, à l'intérieur du boîtier (40) .

12. Dispositif de mélange selon la revendication 11, dans lequel le joint de réglage (70) est disposé entre l'arbre d'entraînement (50) et le boîtier (40) et présente une zone d'étanchéité (71) mobile.

13. Dispositif de mélange selon la revendication 11 ou 12, dans lequel le boîtier (40) présente sur une extrémité d'entraînement du passage (10) un renforcement (90).

14. Dispositif de mélange selon l'une quelconque des revendications précédentes, comprenant au moins un joint permanent (65 ; 66) supplémentaire, qui étanchéifie le passage (10) indépendamment de l'état de fonctionnement d'étanchéité du joint de réglage (70).

15. Dispositif de mélange selon l'une quelconque des revendications précédentes, comprenant l'entraînement (120) disposé à l'extérieur du contenant de mélange (100) pour entraîner l'arbre d'entraînement (50) et/ou le contenant de mélange (100), à l'intérieur duquel le fluide et/ou la matière solide peut être disposé(e).

16. Dispositif de mélange selon l'une quelconque des revendications précédentes, comprenant le contenant de mélange (100), à l'intérieur duquel le fluide et/ou la matière solide peut être disposé(e), dans lequel
- le contenant de mélange (100) présente une paroi de contenant (101) au moins en partie flexible et/ou
- dans lequel le contenant de mélange (100) est réalisé comme un sachet de bioréacteur et/ou sachet de mélange.

17. Procédé pour faire fonctionner un dispositif de mélange selon l'une quelconque des revendications précédentes pour mélanger un fluide et/ou une matière solide avec les étapes :
- de réception du fluide et/ou de la matière solide dans un contenant de mélange (100) ;
- de mélange du fluide et/ou de la matière solide disposé(e) dans le contenant de mélange (100) au moyen d'un élément agitateur (30) disposé au moins en partie à l'intérieur du contenant de mélange (100) ;
- d'entraînement de l'élément agitateur (30) au moyen d'un arbre d'entraînement (50), lequel est entraîné par un entraînement (120) disposé à l'extérieur du contenant de mélange (100) et lequel est guidé sur un passage (10) à travers une paroi de contenant (101) du contenant de mélange (100) ; et
- de réglage d'un joint de réglage (70) réglable pour étanchéifier le passage (10) dans un d'au moins deux états de fonctionnement d'étanchéité avec différents effets d'étanchéité.

18. Procédé selon la revendication 17, dans lequel
- le joint de réglage (70) dans un état d'étanchéité est réglé comme premier état de fonctionnement d'étanchéité, dans lequel le joint de réglage (70) étanchéifie le passage (10), en particulier lorsque l'arbre d'entraînement (50) n'est pas entraîné ; et
- le joint de réglage (70) dans un état d'ouverture est réglé comme deuxième état de fonctionnement d'étanchéité, dans lequel il n'étanchéifie pas et/ou réduit le passage (10), en particulier lorsque l'arbre d'entraînement (50) est entraîné.
